# EUROPEAN PATENT SPECIFICATION

(11) **EP 1 965 810 B1**
(45) Date of publication and mention of the grant of the patent: **17.08.2011**
(21) Application number: 06824296.5
(22) Date of filing: 07.12.2006
(51) Int. Cl.: A61K 33/08, A61P 3/02

(54) **PREPARATION FOR TREATMENT OF MINERAL DEFICIENCY**
ZUBEREITUNG ZUR BEHANDLUNG VON MINERALSTOFFMANGEL
PRÉPARATION POUR LE TRAITEMENT D'UNE CARENCE EN MINÉRAUX

(30) Priority: 07.12.2005 EP 05077820
(43) Date of publication of application: 10.09.2008
(73) Proprietor: Nederlandse Organisatie voor Toegepast -Natuurwetenschappelijk Onderzoek TNO, 2628 VK Delft (NL)
(72) Inventor: SLAGHEK, Theodoor Maximiliaan, 3042 HT Rotterdam (NL); BATENBURG, Lawrence Fabian, 5611 CK Eindhoven (NL); FISCHER, Hartmut Rudolf, 5731 TP Mierlo (NL); JETTEN, Jan Matthijs, 3701 JL Zeist (NL)
(74) Representative: Hatzmann, Martin
(86) International application number: PCT/NL2006/000620
(87) International publication number: WO 2007/067043

(56) References cited:
- EP-A- 1 338 284
- WO-A-94/10972
- WO-A-94/14409
- US-A- 5 641 813
- US-A1- 4 296 094
- US-A1- 2004 052 849
- US-A1- 2006 013 893
- US-B1- 6 852 670
- DATABASE WPI Week 200426 Derwent Publications Ltd., London, GB; AN 2004-274901 XP002425720 & JP 2004 091421 A (TAYCA CORP) 25 March 2004 (2004-03-25)
- KHAN A I ET AL: "Intercalation and controlled release of pharmaceutically active compounds from a layered double hydroxide" CHEMICAL COMMUNICATIONS - CHEMCOM, ROYAL SOCIETY OF CHEMISTRY, GB, no. 22, 21 November 2001 (2001-11-21), pages 2342-2343, XP002367498 ISSN: 1359-7345
- MEYN M ET AL: "ANION-EXHANGE REACTIONS OF LAYERED DOUBLE HYDROXIDES" INORGANIC CHEMISTRY, AMERICAN CHEMICAL SOCIETY, EASTON, US, vol. 29, 1990, pages 5201-5207, XP008004649 ISSN: 0020-1669
- PATENT ABSTRACTS OF JAPAN vol. 1998, no. 09, 31 July 1998 (1998-07-31) & JP 10 101569 A (EISAI CO LTD), 21 April 1998 (1998-04-21)

## Description

The invention relates to the use of a layered double hydroxide as a taste-masking agent in preparations for treatment of mineral deficiency and to preparations having improved efficacy.

The term mineral deficiency means a condition where the concentration of any one of the minerals essential to human and/or animal health is abnormally low in the body. In some cases, an abnormally low mineral concentration is defined as that which leads to an impairment in a function dependent on the mineral. In other cases, the convention may be to define an abnormally low mineral concentration as a level lower than that found in a specific healthy population.

The mineral content of the body may be measured by testing samples of blood plasma, red blood cells, or urine. In the case of calcium and phosphate deficiency, the diagnosis may also involve taking X-rays of the skeleton. In the case of iodine deficiency, the diagnosis may include examining the patient's neck with the eyes and hands. In the case of iron deficiency, the diagnosis may include the performance of a stair-stepping test by the patient. Since all the minerals serve strikingly different functions in the body, the tests for the corresponding deficiency are markedly different from each other.

The class of mineral nutrients typically includes all inorganic elements or inorganic molecules that are required for life. As far as human nutrition is concerned, the inorganic nutrients include water, sodium, potassium, chloride, calcium, phosphate, sulfate, magnesium, iron, copper, zinc, manganese, iodine, selenium, and molybdenum. Some of the inorganic nutrients, such as water, do not occur as single atoms, but occur as molecules. Other inorganic nutrients that are molecules include phosphate, sulfate, and selenite. There is some evidence that other inorganic nutrients, such as chromium and boron, play a part in human and animal health, but their role is not well established. Fluoride has been proven to increase the strength of bones and teeth, but there is little or no reason to believe that it is needed for human and animal life.

Iron is an essential component of hemoglobin, the oxygen carrying complex (pigment) in the blood. Iron is normally obtained through the food in the diet. Iron-deficient people tire easily because their bodies are starved for oxygen. Iron is also part of myoglobin. Myoglobin helps muscle cells store oxygen. Without enough iron, the body's fuel cannot be properly synthesized. Iron Deficiency Anemia (also called IDA) is a condition where a person has inadequate amounts of iron to meet body demands. It is a decrease in the amount of red cells in the blood caused by having too little iron. IDA is usually caused by a diet insufficient in iron or from blood loss. Blood loss can be acute as in hemorrhage or trauma or long term as in heavy menstruation. Iron deficiency anemia is the most common form of anemia. About 20% of women, 50% of pregnant women, and 3% of men are iron deficient.

Treatment of mineral deficiency typically, in less severe cases, entails prescription of special diets based on intake of foods which have a high content of the deficient mineral. In more severe cases, treatment usually includes administration of food supplements. These may take the form of so-called multivitamin or multimineral supplements, containing many vitamins and/or minerals, typically in the Recommended Dietary Allowance (which may also be referred to as Recommended Daily Allowance, RDA). However, mineral supplements containing only one, or a few, specific minerals are also commonly prescribed. For instance, iron supplements can be taken over several months to increase iron levels in the blood. Iron supplements can cause irritation of the stomach and discoloration of bowel movements.

A disadvantage of many mineral supplements is that they produce an unpleasant taste, either during or immediately after oral administration. For some minerals this may be a bitter taste, for others a metallic taste. Many mineral supplements have the form of tablets or capsule since it provides for easy and cost-effective administration. Upon oral administration of such dosage forms, the unpleasant taste may be detected when the bad tasting ingredient comes into contact with the taste buds, particularly by overlong holding the dosage form in the mouth, by inadvertent chewing, or by some other release of the bad tasting ingredient.

Patients give many different reasons for their poor compliance with administration regimen of drugs, including mineral supplements. Examples are unattractive appearance, overlarge size, bad taste or fear that an unchewed dosage form may get stuck in the throat. Patients who have difficulties with oral dosage forms often exhibit a gag reflex which prevents adequate oral administration. This phenomenon occurs more often, but not exclusively, in children.

Although chewable dosage forms have been developed which may overcome some of these problems, many pharmaceutical agents have such a bad taste, e.g. bitter or metallic, that it cannot be tolerated when chewed. The prolonged exposure of the patient to the unpleasant taste may also cause compliance problems. Conventionally, attempts have been made to address this issue by incorporating sweeteners or flavoring agents as taste masking agents in oral dosage forms. These agents provide a secondary flavor which is intended to overwhelm any unpleasant taste of the pharmaceutical agent. Unfortunately, for many powerfully bad tasting pharmaceuticals this approach is inadequate.

US-A-4,296,094 discloses the use of compositions for dental cleaning which comprise as active agents mineral ions in the form of layered double hydroxides.

US 2004/052849 discloses a drug delivery system for the controlled release of a pharmaceutically-active compound by oral route. The system comprises an intercalate of a layered double hydroxide having, before intercalation, layers of metal hydroxides, and having intercalated therein a pharmaceutically-active compound having at least one anionic group.

JP 10 101569 discloses an antacid which possesses neutralization activity for gastric juice and is usable in place of magnesium-aluminum-based antacid. This antacid contains a layer double hydroxide.

In accordance with the present invention, it has surprisingly been found that the unpleasant taste of various cationic minerals can be masked in a highly effective manner by incorporating the mineral into a layered double hydroxide. The invention thus provides the use of a layered double hydroxide as a taste-masking agent in preparations for treatment of mineral deficiency in a patient in need thereof, said preparation comprising a layered double hydroxide into which one or more minerals are incorporated as defined in the claims. Also, the present invention discloses a method for improving the taste of a preparation for treating a condition of mineral deficiency in a human or animal comprising incorporating said mineral into a layered double hydroxide.

By incorporating a mineral into a layered double hydroxide, it has been found possible to effectively mask the bad taste of even such notorious minerals as iron. As a result, patient compliance may be increased significantly by prescription of a preparation as described herein, thereby increasing effective uptake of the deficient mineral. Other advantages of the invention will become clear from the following detailed description of the various embodiments of the invention.

Layered double hydroxides are clays of an anionic nature which may be found in nature or prepared synthetically. They consist of small crystalline sheets of dimensions of a few nanometers up to a few tens of nanometers, between which anions are located. By these anions are meant anions other than hydroxyl groups. For a description of possible methods of preparation for a synthetic layered double hydroxide, reference is made to U.S. Patents 3,539,306 and 3,650,704.

A layered double hydroxide into which one or more minerals are incorporated preferably has the formula

[(M^{II})₍₁₋ₓ₎ (M^{III})ₓ (OH)₂] [A_{x/y}^{y-}.n H₂O] (I)

wherein M^{II} and M^{III} are respectively one or more mono-, bi- and tri-valent metal cations.

In formula (I), M^{II} can be one or more bivalent metals, and M^{III} can be one or more trivalent metals, where x is a number between 0.15 and 0.5, y is 1 or 2, n is a number from 1 to 10, and A is an anion selected from the group consisting of Cl⁻, Br⁻, NO₃⁻, PO₄³⁻, SO₄²⁻, CO₃²⁻, and combinations thereof.

The bivalent cation is preferably selected from the group of bivalent magnesium, chromium, zinc, germanium, nickel, iron, copper, cobalt, calcium, molybdenum, vanadium and manganese ions and combinations of these bivalent cations. The trivalent cation is preferably selected from the group of trivalent aluminum, chromium, iron, cobalt, molybdenum, vanadium and manganese ions and combinations of these trivalent cations.

In a preferred embodiment, the bivalent cation (M^{II})₍₁₋ₓ₎ is a combination of magnesium and a bivalent mineral in which an intended patient is deficient. The stoichiometric ratio of magnesium to the bivalent deficient mineral is preferably between 1:0 and 0:1, more preferably between 0.5:0.5 and 0.1:0.9. Likewise, the trivalent cation (M^{III})ₓ is preferably a combination of aluminum and a trivalent mineral in which an intended patient is deficient. The stoichiometric ratio of aluminum to the trivalent deficient mineral is preferably between 1:0 and 0: 1, more preferably between 0.5:0.5 and 0.1:0.9.

The mono-valent cation is preferably selected from the group of mono-valent lithium, sodium, potassium, and silver and combinations of these mono-valent cations. The trivalent cation is preferably selected from the group of trivalent aluminum, chromium, iron, cobalt, molybdenum, vanadium and manganese ions and combinations of these trivalent cations.

It is possible to treat both animals and humans for a mineral deficiency. It is preferred, however, that humans are treated.

A layered double hydroxide having the formula (I), as described above, may be prepared analogous to any known convenient manner for preparing layered double hydroxides. An example of a suitable method is derived from the method disclosed in US patent 3,639,30C, wherein hydrotalcite having the formula Mg₆Al₂(OH)₁₆CO₃.4H₂O is prepared by mixing an aluminum component with a magnesium component in an aqueous medium in the presence of carbonate ions at a suitable pH, preferably at least 8, and recovering the resultant precipitate, wherein the aluminum component and the magnesium component have suitable counter ions to obtain the desired anion A in the layered double hydroxide. In accordance with the invention, the aluminum component and/or the magnesium component may completely or partially be replaced by an appropriate component of the desired trivalent and/or bivalent cation, respectively, to yield a layered double hydroxide having formula (I).

A preparation as described herein can be used for incorporation into medicinal compositions, such as pharmaceutical compositions or drugs, food supplements, or food or feed products. In these applications, a preparation as described herein will typically be combined with other ingredients typically employed in such products.

When the preparation is used in a medicinal composition or a food supplement, this composition or supplement preferably comprises, in addition to the above described layered double hydroxide, a pharmaceutically acceptable excipient. Depending on the oral dosage form, the excipient may be chosen from the classes of fillers, lubricants, sweeteners, flavorants, colorants, antioxidants, coating materials, and other excipients known to the person skilled in the art, and combinations thereof.

When a preparation as described herein is used in a food or feed product, it will preferably be mixed with the other ingredients of the end product in such a way to provide a substantially homogenous incorporation of the preparation as described herein in the product.

In a preferred embodiment, in particularly wherein a bivalent cationic mineral, which may exist in bivalent or trivalent form (such as iron) is to be administered, a preparation as described herein also comprises an antioxidant. Preferred examples of suitable antioxidants include, but are not limited to, ascorbic acid (vitamin C), tocopherol and tocotrienol (vitamin E), flavonoids, carotenoids such as lycopene and beta-carotene, oxycarotenoids such as zeaxanthin and lutein, BHA (butylated hydroxyanisole), BHT (butylated hydroxytoluene), lecithin and chelating agents, as used in iron (both ferri and ferro) containing fruit or fruit like juices or iron based supplements applicable for anaemia like dextran, dextransulphate, carboxydextran, fumarate, and gluconate and the like. The presence of an antioxidant may help preventing undesired oxidation of bivalent cations to trivalent cations. This is particularly preferred for treating iron deficiencies, wherein iron(II) is much more efficacious than iron(III). In accordance with this embodiment, the antioxidant is preferably present in an amount of 0.001 to 0.5 wt.%, based on the weight of the end product e.g. fruit-like juice or iron based supplement.

In preferred embodiment the bivalent cation and the antioxidant are in a molar ratio that allows an effective prevention of oxidation of the bivalent cation. For example, in the case of iron(II) and ascorbic acid the weight ratio is preferably between 0.03: 1 and 5:1, more preferably between 0.1:1 and 3:1.

Preferably, when applied in a vehicle, the preparation as described herein provides at least 5%, more preferably 100 % and most preferably 1000 % of the recommended daily dosage of the incorporated mineral per 100 mL of vehicle. In case essential vitamins such as ascorbic acid or tocopherol are used as antioxidant, the preparation also provides at least 5 %, more preferably 100 % and most preferably 1000% of the recommended daily dosage of the respective vitamin per 100 mL of vehicle.

Further disclosed are methods for improving the taste of a preparation for treating a condition of mineral deficiency in a human or animal comprising providing said mineral incorporated into a layered double hydroxide having the formula

[(M^{II})₍₁₋ₓ₎ M^{II})ₓ (OH)₂] [A_{x/}y^{y-}.n H₂O] (I)

wherein in formula (I), M^{II} represents one or more bivalent cations, and M^{III} represents one or more trivalent cations, and x is a number between 0.15 and 0.5, y is 1 or 2, n is a number from 1 to 10, and A is an anion selected from the group consisting of Cl⁻, Br⁻, NO₃⁻, PO₄³⁻, SO₄²⁻, CO₃²⁻, and combinations thereof.

In a preferred embodiment of such a method, the mineral deficiency is a deficiency of a cationic mineral, and one or more of said cations in the preparation provide the cationic mineral in which an intended patient is deficient. In further preferred embodiments, the cationic mineral is chosen from the group consisting of calcium, chromium, germanium, iron, cobalt, copper, manganese, molybdenum, vanadium and zinc cations.

In yet other preferred embodiments, said method further comprises incorporating an antioxidant in said layered double hydroxide.

In still other preferred embodiments antioxidant is selected from the group consisting of ascorbic acid (vitamin C), tocopherol and tocotrienol (vitamin E), flavonoids, carotenoids such as lycopene and beta-carotene, oxycamtenoids such as zeaxanthin and lutein, BHA (butylated hydroxyanisole), BHT (butylated hydroxytoluene), lecithin and chelating agents such as dextran, dextransulphate, carboxydextran, fumarate, and gluconate.

The present invention further describes a a method for producing a preparation for treating a condition of mineral deficiency in a human or animal, said method comprising the step of preparing a layered double hydroxide having the formula

[(M^{II})₍₁₋ₓ₎ (M^{III})ₓ (OH)₂] [Ax/^{y-}.n H₂O] (I)

wherein in formula (I), M^{II} represents one or more bivalent cations, and M^{III} represents one or more trivalent cations, and x is a number between 0.15 and 0.5, y is 1 or 2, n is a number from 1 to 10, and A is an anion selected from the group consisting of Cl⁻, Br, NO₃⁻, PO₄³⁻,SO₄²⁻, CO₃²⁻, and combinations thereof;
and either performing an anion exchange reaction wherein anions A in said layered double hydroxide are exchanged for an antioxidant; or preparing said layered double hydroxide by in situ synthesis in the presence of said antioxidant.

In preferred embodiments of such a method the mineral deficiency is a deficiency of a cationic mineral, and one or more of said cations in said preparation provide the cationic mineral in which an intended patient is deficient.

Preferably the antioxidant is selected from the group consisting of ascorbic acid (vitamin C), tocopherol and tocotrienol (vitamin E), flavonoids, carotenoids such as lycopene and beta-carotene, oxycarotenoids such as zeaxanthin and lutein, BHA (butylated hydroxyanisole), BHT (butylated hydroxytoluene), lecithin and chelating agents such as dextran, dextransulphate, carboxydextran, fumarate, and gluconate.

In other preferred embodiments of the method for producing a preparation for treating a condition of mineral deficiency in a human or animal, the anion is selected from Cl⁻, Br-, NO₃⁻, PO₄³⁻, SO₄²⁻, and combinations thereof, and the exchange is preformed under conditions which prevent the exchange of CO₃²⁻.

In highly preferred embodiments, the antioxidant is ascorbic acid.

Further disclosed is a preparation for treating a condition of mineral deficiency in a human or animal, obtainable by a method for producing such a preparation fo as described above.

Further disclosed is a preparation for treating a condition of mineral deficiency in a human or animal, said preparation comprising a layered double hydroxide having the formula

[M^{II})₍₁₋ₓ₎ (M^{III})ₓ (OH)₂] [A_{x/y}^{y-}.n H₂O] (I)

wherein in formula (I), M^{II} represents one or more bivalent cations, and M^{III} represents one or more trivalent cations, and x is a number between 0.15 and 0.5, y is 1 or 2, n is a number from 1 to 10, and A is an anion selected from the group consisting of Cl⁻, Br⁻, NO₃⁻, PO₄³⁻, SO₄²⁻, CO₃²⁻, and combinations thereof; and
said preparation further comprising an antioxidant incorporated in said layered double hydroxide. Preferably, in such a preparation, the amount of the layered double hydroxide having incorporated therein the antioxidant, is at least 70 wt.%, more preferably at least 80 wt.%, more preferably at least 90 wt%, still more preferably at least 95 wt.%, most preferably 100%, based on the total weight of the preparation. Preferably, the mineral deficiency is a deficiency of a cationic mineral, and one or more of said cations in the preparation provide the cationic mineral in which an intended patient is deficient. The cationic mineral in a preparation of the invention preferably selected from the group consisting of calcium, chromium, germanium, iron, cobalt, copper, manganese, molybdenum, vanadium and zinc cations, most preferably iron or zinc. The most preferred antioxidant is ascorbic acid.

The invention will now be elucidated by the following, non-restrictive examples.

### Example 1. Synthesis of Mg-Fe(III)-Cl LDH in a Fe:Mg ratio of 4:1

75.12 g of NaOH was weighed under nitrogen atmosphere in a 5 L three-necked flask. Then, 937 mL of boiled demineralized water (cooled to about 40 °C) was added under nitrogen atmosphere. 141.75 g of MgCl₃ · 6 H₂O was weighed in a beaker and dissolved in 875 g of boiled demineralized water (cooled) after which 47.05 g of FeCl₃ · 6 H₂O was added and the mixture was stirred under nitrogen. The brown solution was added to a dropping funnel and fixed to the 5 L three-necked flask in a nitrogen atmosphere. While stirring intensively the Mg-Fe solution was added dropwise until the reaction mixture had a pH of 9.5. Then, the solution was heated for 6 hours at 100 °C. The solution was filtered and washed with demineralized water until all NaCl had been removed. The product was freeze-dried.

### Example 2. Synthesis of Mg-Fe(III)-Cl LDH in a Fe:Mg ratio of 2:1

11.56 g of NaOH was weighed under nitrogen atmosphere in a 500 mL three-necked flask. Then, 145.65 mL of boiled demineralized water (cooled to about 40 °C) was added under nitrogen atmosphere. 19.72 g of MgCl₃ · 6 H₂O was weighed in a beaker and dissolved in 146 g of boiled demineralized water (cooled) after which 13.10 g of FeCl₃ · 6 H₂O was added and the mixture was stirred under nitrogen. The brown solution was added to a dropping funnel and fixed to the 500 mL three-necked flask in a nitrogen atmosphere. While stirring intensively the Mg-Fe solution was added dropwise until the reaction mixture had a pH of 9.5. Then, the solution was heated for 6 hours at 100 °C. The solution was filtered and washed with demineralized water until all NaCl has been removed. The product was freeze-dried.

### Example 3. In-situ synthesis of Mg-Fe(III)-Cl LDH in a Fe:Mg ratio of 4:1 with vitamin C

41.6 g of NaOH was weighed under nitrogen atmosphere in a 2 L glass or steel reactor. Then, 467 g of boiled demineralized water (cooled to about 40 °C) was added under nitrogen atmosphere. Then, 17.6 g of vitamin C was added. 70.2 g of MgCl₃ · 6 H₂O was weighed in a beaker and dissolved in 430 g of boiled demineralized water (cooled) after which 23.4 g of FeCl₃ · 6 H₂O was added and the mixture was stirred under nitrogen. The brown solution was added to a dropping funnel and fixed to the 2 L reactor in a nitrogen atmosphere. While stirring intensively the Mg-Fe solution was added dropwise until the reaction mixture had a pH of 9.5. Then, the solution was heated for 6 hours at 100 °C. The solution was filtered and washed with demineralized water until all NaCl had been removed. The product was freeze-dried.
Analysis by X-ray diffraction, thermal gravimetrical analysis, and infrared spectroscopy showed the presence of vitamin C in the Mg-Fe(III)-Cl LDH

### Example 4 In vitro application of pharmaceutical composition

In duplicate experiments, 1 g of a product prepared according to Example 1 (containing 220 g Fe(III) per kg of hydrotalcite) and 4 g of vitamin C were mixed with artificial saliva and drinking water. The total intake in the gastric compartment of the model was 200 g.

Experiments were performed in TIM (TNO's Intestinal Model). For a discussion of this model, see inter alia the following publications:
Freidig, A. and Verwei, M. (2004). Integration of in vitro data in kinetic models for pharmaceuticals and nutrients. Netherlands Centre Altern. Animal Use Newsletter 16: 1-3.
Havenaar, R. and Minekus, M. (1996). Simulated assimilation. Dairy Industries International 61 (9): 17-23.
Minekus, M. and Havenaar, R. (1998). Reactor system. European Patent No. 0642382. European Patent Bulletin 98/07, Art. 97(4) and (5) EPC, dated 11.02.98.
Minekus, M. and Havenaar, R. (1996). In vitro model of an in vivo digestive tract. United States Patent; nr. 5,525,305, dated June 11, 1996.
Minekus, M., Marteau, P., Havenaar, R. and Huis in 't Veld, J.H.J. (1995). A multi compartmental dynamic computer-controlled model simulating the stomach and small intestine. Alternatives to Laboratory Animals (ATLA) 23: 197-209.
Smeets-Peeters, M.J.E., Watson, T., Minekus, M., Havenaar, R. (1998). A review of the physiology of the canine digestive tract related to the development of in vitro systems. Nutrition Research Reviews 11: 45-69.

These experiments were performed under the average physiological conditions of the gastrointestinal tract as described for humans. These conditions include especially the dynamics of gastric emptying and intestinal transit times, the gastric and the intestinal pH values, and the composition and activity of the secretion products. The digested and dissolved low-molecular compounds are dialysed continuously from the jejunum and ileum compartments of the model via hollow fibre membrane systems. The experiments were performed during 300 minutes, resulting in a dialysate of 5 L. In the dialysate on average 19 mg/L of soluble iron (Fe(II)) was measured (AAS) (atomic absorption spectroscopy).

### Example 5. In vitro application of pharmaceutical composition

A syrup containing 60 gram carbohydrate, 0.3 grams protein, 0 grams fat and 0.5 gram dietary fiber, 100 mg Vitamin C (100 % of the daily intake) and 0.2 gram iron containing hydrotalcite prepared according to Example 1 (220 iron mg/g) was made. 100 ml of the syrup was introduced in the gastric compartment of the TIM Model. The experiments in TIM (TNO's Intestinal Model) were performed under the average physiological conditions of the gastrointestinal tract as described for humans. These conditions include especially the dynamics of gastric emptying and intestinal transit times, the gastric and the intestinal pH values, and the composition and activity of the secretion products. The digested and dissolved low-molecular compounds are dialyzed continuously from the jejunum and ileum compartments of the model via hollow fiber membrane systems. The experiments were performed during 300 minutes, resulting in a dialysate of 5 L. In the dialysate on average 3.1 mg/L of soluble iron was measured (AAS) (atomic absorption spectroscopy). In total 15.5 (51 * 3.1 mg soluble iron per 1) was bioavailable resembling approximately 100 % of the daily iron intake.

### Example 6. Synthesis of Mg-Fe(III)-Cl (Fe:Mg 4:1)

An amount of 75.12g of NaOH was weighed-in into a 5 liter three-necked flask (under N2 atmosphere; flask was flushed with N2/vacuum) and 937ml boiled demineralized water (cooled to ∼40°C) was added (NaOH solon. = 2 M, flushed with N2/vacuum and kept under N2 atmosphere).

An amount of 141.75g of MgCl3.6H2O was weighed into a measuring beaker and dissolved in 875g boiled demineralized water (cooled to ∼40 °C)) and 47.05g FeCl3.6 H2O was added and stirred under N2. The brown solution was added to a dropping funnel and mounted on the neck of the 5 liter three-necked flask. The complete system was flushed with N2/vacuum and kept under N2 atmosphere. With rigorous stirring (top stirrer, level 8-10) and monitoring of the pH in the solution, the Mg-Fe solution was added drop wise to the NaOH solution until a pH of 9.5 was reached. Then the solution was heated at 100°C for 6 hours. Finally, the solution was filtered and washed with demineralized water until all NaCl was removed, and freeze-dried.

### Example 7 Synthesis of Mg-Fe(III)-Cl (Fe:Mg 2:1)

An amount of 11.56g of NaOH was weighed-in into a 500 mL three-necked flask (under N2 atmosphere; flask was flushed with N2/vacuum) and 145.65ml boiled demineralized water (cooled to ∼40 °C)) was added (NaOH soln. = 2 M, flushed with N2/vacuum and kept under N2 atmosphere).

An amount of 19.72g of MgCl3.6H2O was weighed into a measuring beaker and dissolved in 146g boiled demineralized water (cooled to ∼40 °C) and 13.10g FeCl3.6 H2O was added and stirred under N2. The brown solution was added to a dropping funnel and mounted on the neck of the 500 mL three-necked flask. The complete system was flushed with N2/vacuum and kept under N2 atmosphere. With rigorous stirring (top stirrer, level 8-10) and monitoring of the pH in the solution, the Mg-Fe solution was added drop wise to the NaOH solution until a pH of 9.5 was reached. Then the solution was heated at 100°C for 6 hours. Finally, the solution was filtered and washed with demineralized water until all NaCl was removed, and freeze-dried.

### Analysis of Mg-Fe(III) LDHs produced in Example 6 and 7

A preliminary yield after washing and freeze/drying of part of the material produced in Example 6 (4:1) was 35.11 g (46% relative to the starting amount). The product obtained is a light brown (cream-colored) powder. The yield for the material produced in Example 7 (2:1) was 10.23 g (67% relative to the starting amount). The product obtained is a red-brown powder. Both products dissolve in HCl whereby a clear yellow solution is obtained. The presence of small bubbles in this solution indicates the presence of carbonate (CO₃²⁻).

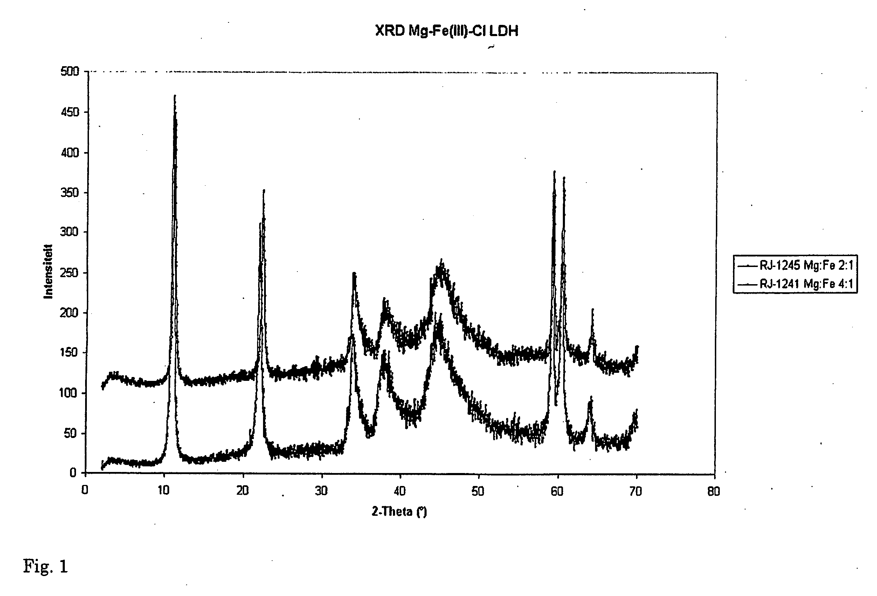

Fig. 1 shows the XRD pattern of the synthesized Mg-Fe(III)-Cl LDHs of Example 6 (4:1) and of Example 7 (2:1). The d-spacing values for the 4:1 and 2:1 Mg-Fe(III)-Cl are 8.08 en 7.98 Å, respectively, and are displayed in Table 1. There is a small shift in d-spacing and 2-Theta from 4:1 to 2:1. A search-match with a database of reference patterns indicates that the synthesized product is Mg4Fe(OH)8OCl.x H2 O (Iowaite).

**Tabel 1: 2-Theta en d-spacing of 2:1 en 4:1**

| **Mg-Fe(III)-Cl 4:1 (Example 6)** | | | | | | | | |
|---|---|---|---|---|---|---|---|---|
| *2-Theta (°)* | 10.94 | 21.97 | 33.92 | 38.13 | 45.07 | 59.19 | 60.38 | 63.93 |
| *d-spacing* | 8.08 | 4.04 | 2.64 | 2.36 | 2.01 | 1.56 | 1.53 | 1.45 |

| **Mg-Fe(III)-Cl 2:1 (Example 7)** | | | | | | | | |
|---|---|---|---|---|---|---|---|---|
| *2-Theta (°)* | 11.08 | 22.35 | 34.05 | 37.98 | 45.00 | 59.32 | 60.54 | 64.20 |
| *d-spacing* | 7.97 | 3.97 | 2.63 | 2.37 | 2.01 | 1.56 | 1.53 | 1.45 |

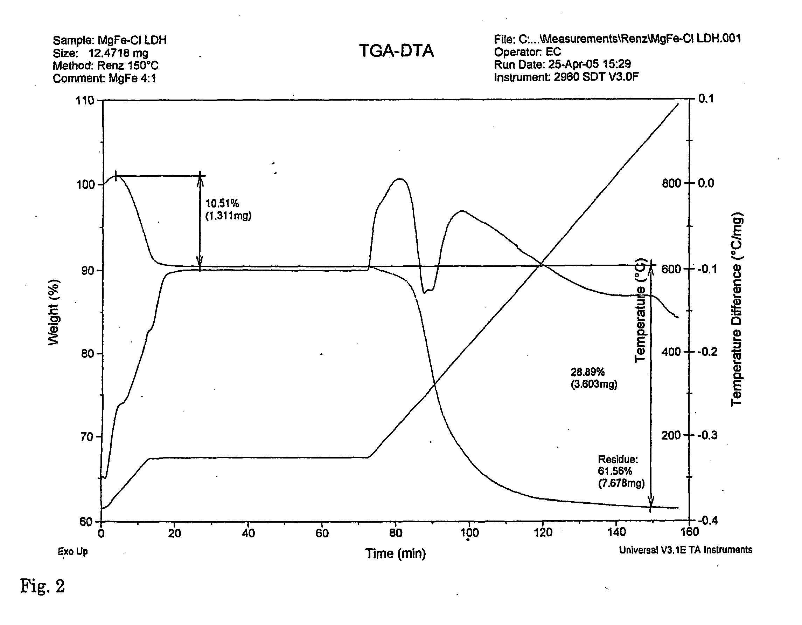

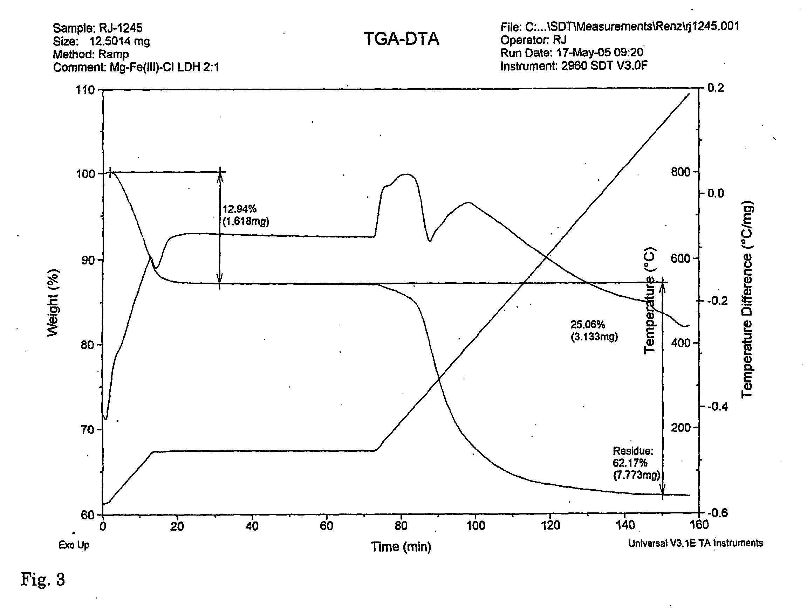

The Tga curves of the materials produced in Examples 6 (4:1) and 7 (2:1) are presented in Figures 2 and 3, respectively. The settings were 10°C/min. to 150°, then 1 hour 150°C, thereafter 10°C/min. to 1000°C.

**Tabel 2: Tga-analysis**

| **Sample** | **% Reduction to 200°C** | **% Reduction to 1000°C** | **% Residu** |
|---|---|---|---|
| Mg-Fe(III)-Cl 4:1 (Example 6) | 10.51 | 28.89 | 61.56 |
| Mg-F (III)-Cl 2:1 (Example 7) | 12.94 | 25.06 | 62.17 |

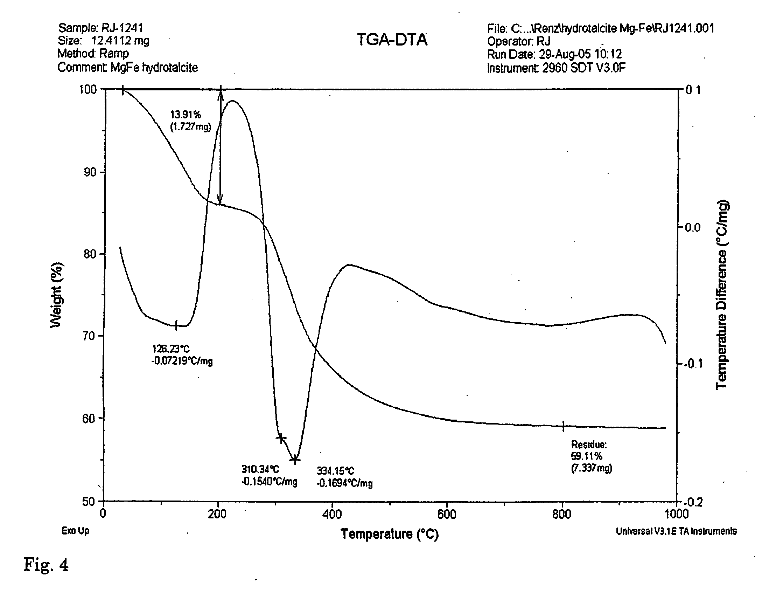

Typically, Mg-Fe(III)-Cl hydrotalcite exhibits three distinct peaks in Tga-analysis: around 135°C for water, and around 350°C and 370°C (dehyclioxylation and loss of volatile species, HCl). These three peaks are present in the Tga curve of the material produced in Example 6 (20°C/min. to 1000°C) which is indicative of an Mg-Fe(III)-Cl hydrotalcite, see Figure 4. Using ICP-analysis the concentration of Fe and Mg was determined in samples of the materials obtained in Examples 6 and 7. The results are provided in Table 3.

**Tabel 3: ICP- analysis**

| **Sample** | **Fe (mg/l)** | **Mg (mg/l)** | **Fe (mol/l)** | **Mg (mol/l)** | **Ratio Mg:Fe (mol/mol)** |
|---|---|---|---|---|---|
| Example 6 (1^{st} sample) | 8.73 | 15.1 | 0.157 | 0.621 | 3.97 |
| Example 6 (2^{nd} sample) | 7.54 | 12.9 | 0.135 | 0.531 | 3.93 |
| Example 7 | 9.33 | 7.57 | 0.167 | 0.312 | 1.87 |

### Example 8 Exchange of Mg-Fe(III)-Cl (Fe:Mg 4:1) with vitamin C under acidic and alkaline conditions.

### A. Under acidic conditions:

An amount of 8 g of vitamin C (L-ascorbic acid, 99%, Aldrich, Fw=176.12g/mol) was dissolved in 250g of demineralized water in a 500 mL three-necked flask. To this was added an amount of 2 g of Mg-Fe(III)-Cl hydrotalcite. The mixture was heated overnight at 80°C in an oil bath under stirring. The pH of the mixture was 4-5.

### B. Under alkaline conditions:

An amount of 8 g of vitamin C (L-ascorbic acid, 99%, Aldrich, Fw=176.12g/mol) was dissolved in 250g of demineralized water in a 500 mL three-necked flask. The solution is alkalinated using a 1 M NaOH solution (50g). To this was added an amount of 2 g of Mg-Fe(III)-Cl hydrotalcite. The mixture was heated overnight at 80°C in an oil bath under stirring. The pH of the mixture was 10. The material was filtered and washed until all Cl- was removed, and was freeze-dried.

### Results of exchange reaction.

Exchange of hydrotalcite with vitamin C under acidic conditions (RJ-1266A) produces a black solution after 1 night at 80°C. Upon contact between the hydrotalcite and the vitamin C, the color immediately changes to purple and the change in color of the solution progresses in time from purple to black. This material cannot be filtered is it is believed that this is degraded hydrotalcite. The exchange under alkaline conditions produces a brown solution after 1 night with a brown precipitate. Also in this case, the color of the hydrotalcite solution changes to purple immediately upon contact with the vitamin C solution. This material can be filtered and is analyzed using for instance XRD and Tga-analysis.

Figure 4 shows the XRD pattern of the exchanged hydrotalcite.

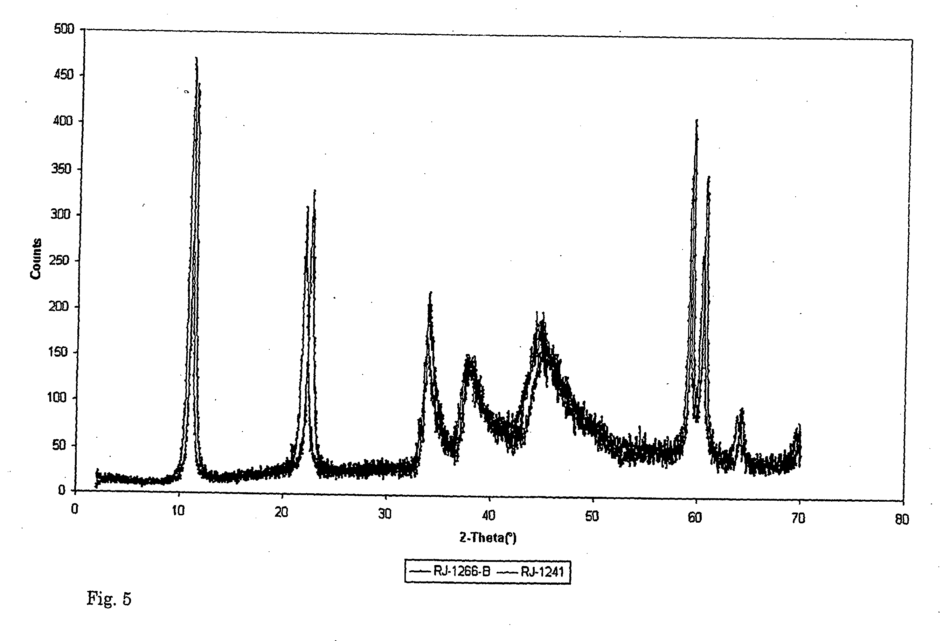

From Figure 5 is can be inferred that a small shift takes place in the direction of a higher 2-Theta(°) which is indicative of a smaller d-spacing. This means that no exchange has occurred between hydrotalcite and vitamin C.

**Tabel 4: 2-Theta(°) and d-spacing**

| **Mg-Fe(III)-Cl 4:1 exchanged with vitamin C (RJ-1266B)** | | | | | | | | |
|---|---|---|---|---|---|---|---|---|
| *2-Theta (*°*)* | 11.23 | 22.53 | 34.06 | 38.41 | 45.53 | 59.36 | 60.62 | 64,31 |
| *d-spacing* | 7.87 | 3.94 | 2.63 | 2.34 | 1.99 | 1.56 | 1.53 | 1.45 |

| **Mg-Fe(III)-Cl 4:1 (RJ-1241)** | | | | | | | | |
|---|---|---|---|---|---|---|---|---|
| *2-Theta (*°*)* | 10.94 | 21.97 | 33.92 | 38.13 | 45.07 | 59.19 | 60.38 | 63.93 |
| *d-spacing* | 8.08 | 4.04 | 2.64 | 2.36 | 2.01 | 1.56 | 1.53 | 1.45 |

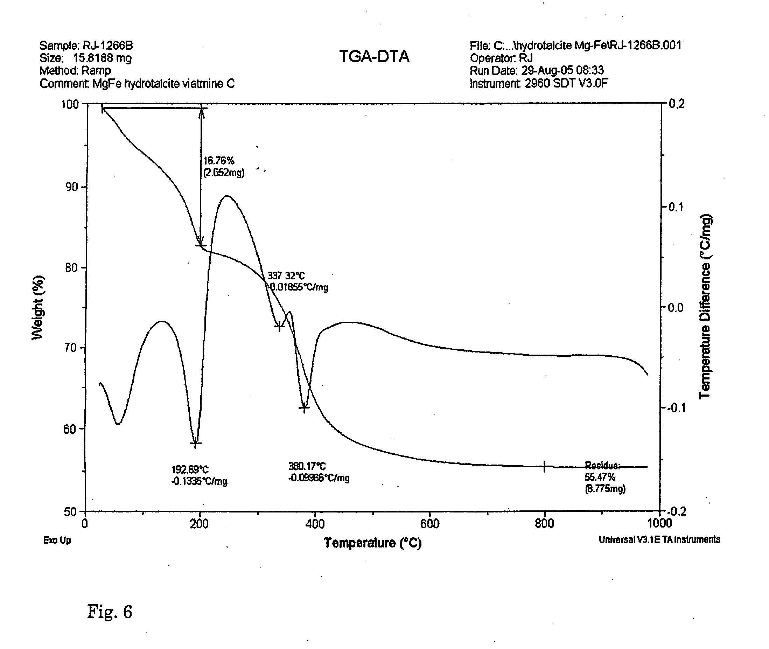

Figure 6 shows the Tga-analysis of the exchanged hydrotalcite.

As described above, the three peaks that are characteristic for the Mg-Fe(III)-Cl hydrotalcite are positioned around 135°C, 350°C and 370°C. These peaks cannot be detected in the Tga of the exchanged hydrotalcite. In this case peaks are visible around 190°C and 380°C. These peaks are indicative of the Mg-Fe(III)-CO3 hydrotalcite.

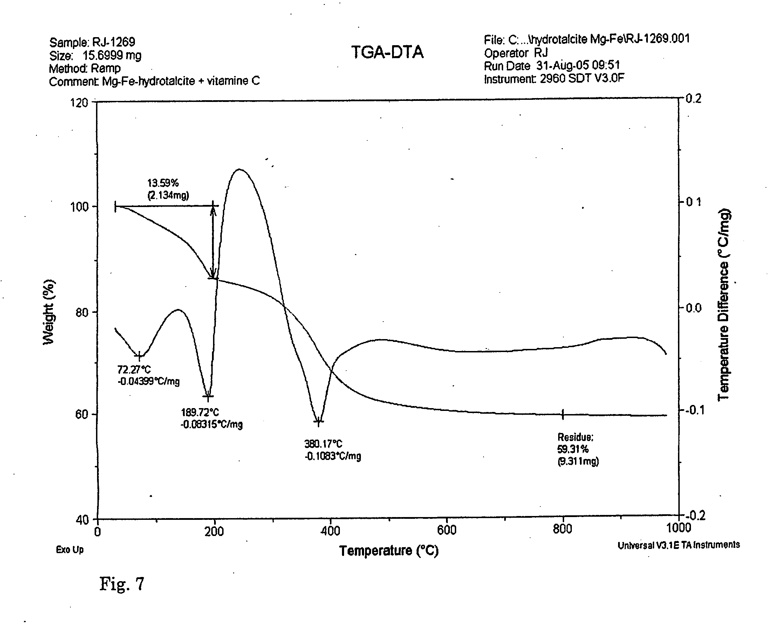

Exchange under nitrogen atmosphere and boiled water provides a Tga analysis result as displayed in Figure 7. Also in this instance peaks are visible at 190°C and 380°C indicative of Mg-Fe(III)-CO3 hydrotalcite.

Apparently, the Mg-Fe(III)-Cl hydrotalcite is converted into the Mg-Fe(III)-CO3 hydrotalcite. Since Cl- is larger than CO3, this may explain the shift in the XRD curve.

### Example 9 In-situ synthesis of Mg-Fe(III)-Cl (Mg:Fe 4:1) with Vitamin C

An amount of 41.6g of NaOH (0.94 mol + 0.1 mol compensation for Vitamin C) was weighed-in into a 2 liter flensch reactor (under N2 atmosphere; flask was flushed with N2/vacuum) and 467 g of boiled demineralized water (cooled to ∼40 °C) was added (pH=13.40) (NaOH solution = 2 M, flushed with N2/vacuum and kept under N2 atmosphere). Then 17.6 g of Vitamin C was added. The color of the solution changes from clear colorless to clear slightly yellow. The solution was flushed with N2/vacuum and kept under N2 atmosphere (pH =13.04).

An amount of 70.2g of MgCl3.6H2O was weighed into a measuring beaker and dissolved in 430g boiled demineralized water (cooled to ∼40 °C) and 23.4g FeCl3.6 H2O was added and stirred under N2. The brown solution was added to a dropping funnel and mounted on the reactor. The complete system was flushed with N2/vacuum and kept under N2 atmosphere. Under constant stirring and monitoring of the pH in the solution, the Mg-Fe solution was added drop wise to the NaOH solution until a pH of 9.5 was reached. Then the solution was heated at 100°C for 6 hours. Finally, the solution was centrifuged and washed with demineralized water until all NaCl was removed, and freeze-dried.

### Results in-situ Mg-Fe(III)-Cl / vitamin C synthesis.

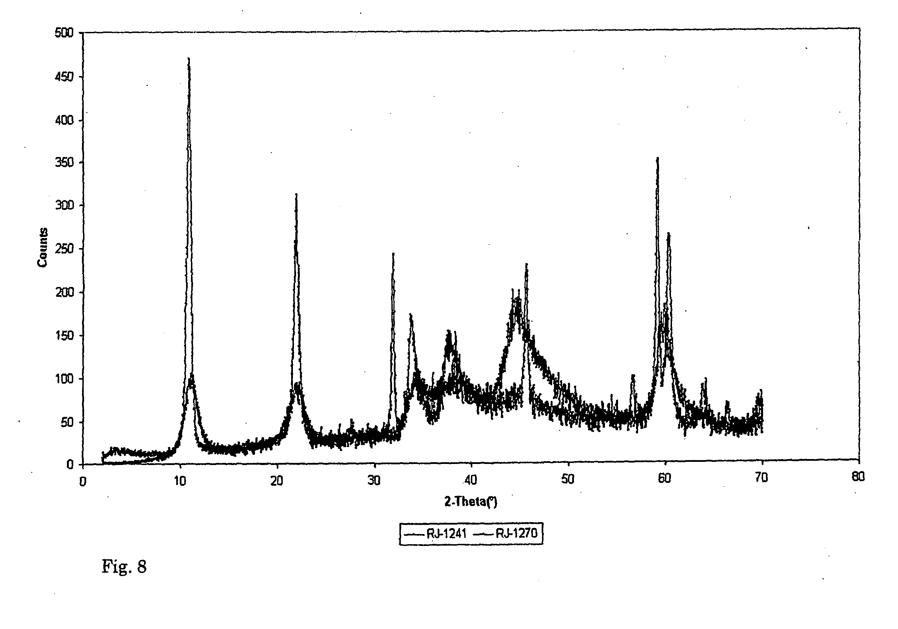

During the reaction the solution changes color to light black. The color of the product obtained after freeze-drying is brown. The XRD pattern is provided in Figure 8 together with the starting material. From the Table and the XRD curve it shows that the in-situ synthesis with vitamin c is slightly shifted towards higher 2-Theta which produces a reduction in d-spacing. However, the peaks are broader and there is an extra peak at a 2-Theta of 31.92°.

**Table 5. 2-Theta(°) and d-spacing**

| **Mg-Fe(III)-Cl 4:1 in-situ synthesis with vitamin C (RJ-1270)** | | | | | | | | |
|---|---|---|---|---|---|---|---|---|
| *2-Theta (°)* | 11.13 | 21.95 | 34.13 | 38.33 | 45.61 | 56.58 | 59.87 | 66.40 |
| *d-spacing* | 7.94 | 4.04 | 2.62 | 2.35 | 1.99 | 1.63 | 1.54 | 1.41 |

| **Mg-Fe(III)-Cl 4:1 (RJ-1241)** | | | | | | | | |
|---|---|---|---|---|---|---|---|---|
| *2-Theta (°)* | 10.94 | 21.97 | 33.92. | 38.13 | 45.07 | 59.19 | 60.38 | 63.93 |
| *d-spacing* | 8.08 | 4.04 | 2.64 | 2.36 | 2.01 | 1.56 | 1.53 | 1.45 |

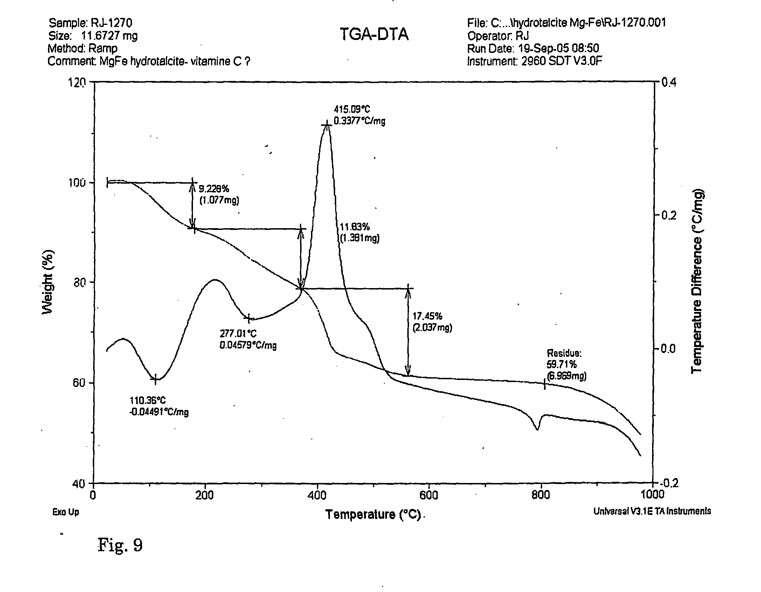

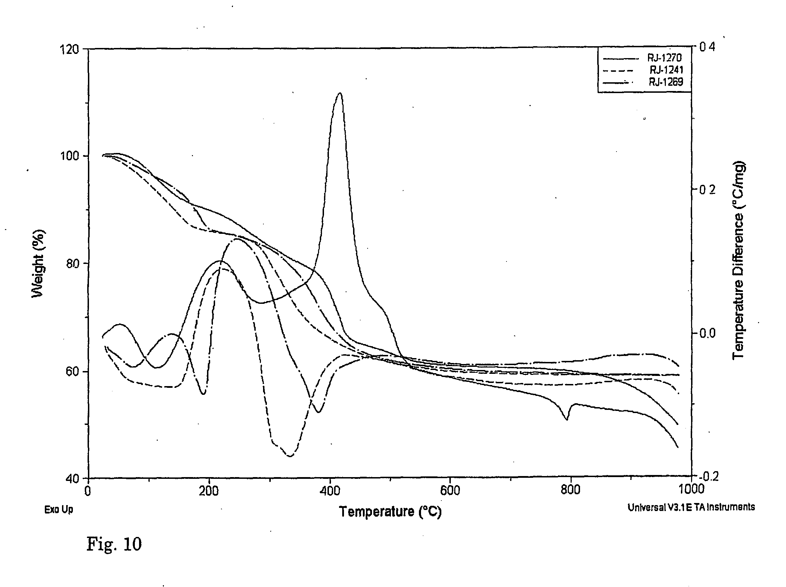

Tga-analysis of the in-situ synthesis with vitamin C is displayed in Figure 9 and in Figure 10 the product is compared with the reference and an exchange reaction.

From Tga-analysis it is clear that an extra peak is present at 415°C, which is not present in the starting material (reference product) or the product of the exchange reaction. This exothermal peak coincides with a weight loss of 17%.

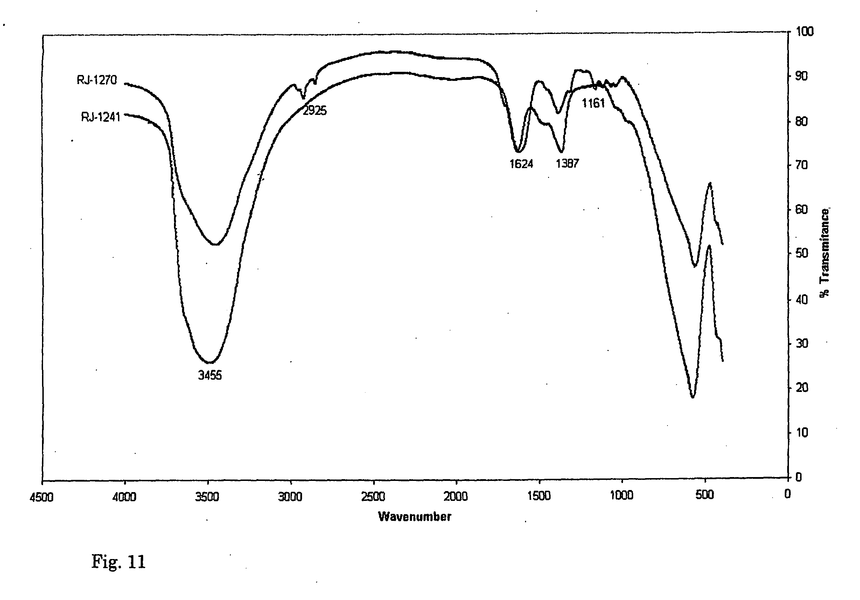

The presence of Vitamin C was assessed using IR. The IR spectrum is presented in Figure 11 together with the reference material. In the case of in situ synthesis with vitamin C extra peaks appear around 2925 and 1161. The intensity of the peak at 1624 is also higher than in the reference material and a shoulder is visible between 1700-1800, which is not present in the reference material.

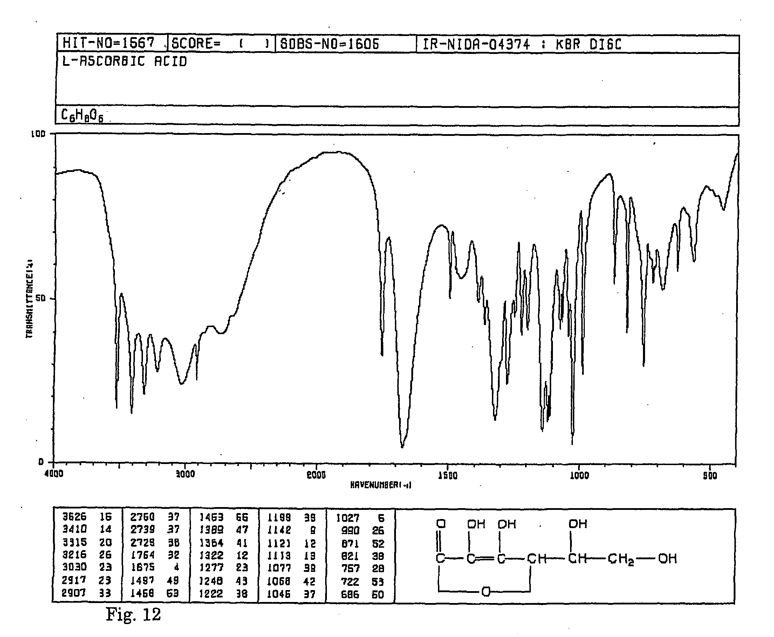

The IR spectrum of Vitamin C is displayed in Figure 12. Here, the peaks around 2925 and 1161 are clearly visible. Also, the peak around 1600 with shoulder is visible.

### Example 10 Synthesis of Mg-Al-CO3 LDH at pH 7.86

The following method was adapted from Applied Catalysis A: General 287 (2005) 183-190, "Structure correlations in MgAl hydrotalcite catalysts for biodiesel synthese". A solution of 51.45g Mg(NO3)2.6H2O and 37.56g Al(NO3)3.9H2O in 201.71g demineralized water and a solution of 38.7g (NH4)2CO3 in 200.37g demiwater were added drop wise (dropping funnel) over a period of 1 hour to an amount of 200ml demineralized water at 65°C under stirring. During this procedure, the pH was continuously kept at pH 7.86 using a 35% NH40H solution and a pHstat. After the addition, the solution is maintained at 65°C for 3 hours under stirring. Then, the solution is filtered and washed to pH 7 and dried at 118°C for 18 hours.

### Example 11 Synthesis of Mg-Al-CO3 LDH at pH 7.86

Using a dosimat (2.5ml/min.), a solution of 128.28g of Mg(NO3)2.6H2O and 94g of Al(NO3)3.9H2O in 500g of demineralized water and an amount of 150 ml of a solution of 96.20g (NH4)2CO3 in 500g demineralized water were added drop wise to an amount of 75ml of demineralized water at 55°C under stirring. During this procedure, the pH was continuously kept at pH 7.86 using a 35% NH4OH solution and a pHstat. After the addition, the solution is maintained at 65°C for 3 hours under stirring. Then, the solution is filtered and washed to pH 7 and dried at 118°C for 18 hours.

### Results synthesis Mg-Al-CO3 LDH at pH 7.86.

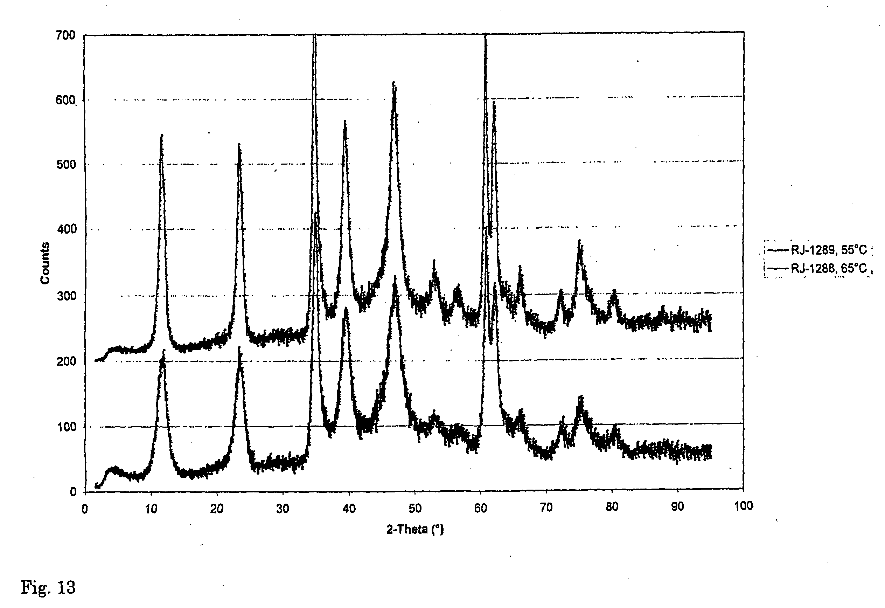

The reflection values (2-Theta) measured for the synthesized Mg-Al-CO3- LDH correspond to those provided as reference in the literature (Weiqing Meng et al., Material Research Bulletin 39 (2004) 1185-1193, see Table 6 en Figure 13).

**Table 6: 2-Theta(°) values for Mg-Al-CO3- LDH compared to products synthesized in Examples 10 and 11.**

| **LDH ref** (Weiqing Meng *et al*.) | **Example 10** | **Example 11** |
|---|---|---|
| 11.6 | 11.66 | 11.76 |
| 23.4 | 23.45 | 23.41 |
| 35 | 34.87 | 35.02 |
| 39.6 | 39.46 | 39.51 |
| 47.1 | 46.87 | 46.93 |
| 53.4 | 52.56 | 53.21 |
| 56.8 | 57 | 56.87 |
| 61.1 | 60.7 | 60.94 |
| 62.4 | 62.15 | 62.24 |

### Example 12 In-vitro model of the gastrointestinal Tract (TNO-TIM model)

In duplicate experiments, 1 gram of clay as produced in Example 9 (RJ-1270), containing iron III and vitamin C, was mixed with artificial saliva and drinking water. The total intake in the gastric compartment of the model was 200 g.

The experiments in the TIM model as described in Example 4 were performed under the average physiological conditions of the gastrointestinal tract as described for humans. These conditions include especially the dynamics of gastric emptying and intestinal transit times, the gastric and the intestinal pH values; and the composition and activity of the secretion products. The digested and dissolved low-molecular compounds are dialysed continuously from the jejunum and ileum compartments of the model via hollow fibre membrane systems. The experiments were performed during 300 minutes, resulting in a dialysate of 5 1. In the dialysate on average 5 mg/l of soluble iron (Fe(II))was measured (AAS).

### Legend to the Figures

Figure 1: XRD analysis of the material produced in Example 6 (4:1) and Example 7 (2:1).
Figure 2: Tga analysis of the material produced in Example 6 (4:1).
Figure 3: Tga analysise of the material produced in Example 7 (2:1).
Figure 4: Tga analysis of the material produced in Example 6.
Figure 5: XRD analysis of material of Example 6 (starting material; RJ-1241) and material of Example 8 (exchanged hydrotalcite under alkaline conditions; RJ-1266B).
Figure 6: Tga analyse of material of Example 8 (exchanged hydrotalcite under alkaline conditions)
Figure 7: Tga analysis of material of Example 8 (exchanged hydrotalcite under alkaline conditions)
Figure 8: Xrd analysis of material from Example 6 (RJ-1241) and Example 9 (RJ-1270)
Figure 9: Tga analysis of material from Example 9
Figure 10: Tga analysis of Material from Example 6 (RJ-1241), Example 8 (exchange under alkaline conditions; RJ-1269), and Example 9 (RJ-1270) Figure 11: IR analysis of material from Example 6 (RJ-1241) and Example 9 (RJ-1270)
Figure 12: IR analysis of Vitamin C
Figure 13: Xrd analysis of material from Example 11 (RJ-1289).

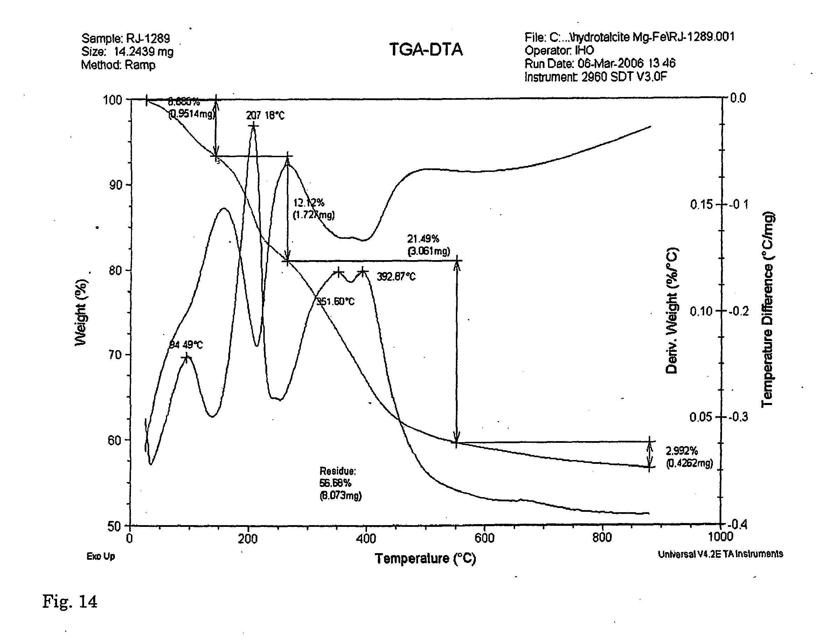

Figure 14. Tga-analysis of material of Example 11 (RJ-1289). The Tga analysis of the material of Example 10 was similar to this.

## Claims

1. Preparation for treatment of a deficiency of a cationic mineral, said preparation comprising a layered double hydroxide having one or more cationic minerals, wherein the layered double hydroxide has the formula
[(M^{II})₍₁₋ₓ₎ (M^{III})ₓ (OH)₂] [A_{x/y}^{y-}·n H₂O] (I),
wherein in formula (I), M^{II} represents one or more bivalent cations, and M^{III} represents one or more trivalent cations, and x is a number between 0.15 and 0.5, y is 1 or 2, n is a number from 1 to 10, and A is an anion selected from the group consisting of Cl⁻, Br⁻, NO₃⁻, PO₄³⁻, SO₄²_{'}, CO₃²-, and combinations thereof.

2. Preparation according to claim 1, wherein the cationic mineral is chosen from the group consisting of calcium, chromium, germanium, iron, cobalt, copper, manganese, molybdenum, vanadium and zinc cations.

3. Preparation according to claim 1 or 2, wherein the bivalent cation is selected from the group of bivalent magnesium, chromium, zinc, germanium, nickel, iron, copper, cobalt, calcium, molybdenum, vanadium and manganese ions and combinations of these bivalent cations.

4. Preparation according to any of the preceding claims, wherein the trivalent cation is selected from the group of trivalent aluminum, chromium, iron, cobalt, molybdenum, vanadium and manganese ions and combinations of these trivalent cations.

5. Preparation according to any one of claims 3 or 4, wherein one or more of said cations in said preparation provide the cationic mineral in which an intended patient is deficient.

6. Preparation according to claim 5, wherein M^{II} represents a combination of magnesium and a bivalent mineral in which an intended patient is deficient.

7. Preparation according to claim 6, wherein the stoichiometric ratio of magnesium to the bivalent deficient mineral is between 1:0 and 0:1.

8. Preparation according to claim 7, wherein M^{III} represents a combination of aluminum and a trivalent mineral in which an intended patient is deficient.

9. Preparation according to claim 8, wherein the stoichiometric ratio of aluminum to the trivalent deficient mineral is between 1:0 and 0:1.

10. Preparation according to any one of the preceding claims, further comprising an antioxidant for preventing undesired oxidation of said bivalent cations to trivalent cations.

11. Preparation according to any one of the preceding claims, wherein the bivalent cation is Fe²⁺.

12. Preparation according to any claim 10 or 11, wherein said antioxidant is incorporated in said layered double hydroxide.

13. Preparation according to any one of claims 10-12, wherein said antioxidant is selected from the group consisting of ascorbic acid (vitamin C) and chelating agents.

14. Food supplement, food or feed product for treatment of a deficiency of a cationic mineral comprising the preparation according to any one of claims 1-13.

15. Medicinal composition for treatment of a deficiency of a cationic mineral comprising the preparation according to any one of claims 1-13.

## Patentansprüche

1. Zubereitung zur Behandlung eines Mangels an kationischem Mineral, wobei die Zubereitung Schicht-Doppelhydroxid mit einem oder mehreren kationischen Mineral(ien) umfasst, wobei das Schicht-Doppelhydroxid die Formel
[(M^{II}) ₍₁₋ₓ) (M^{III})ₓ (OH)₂] [A_{x/}y^{y-}·nH₂O] (I)
hat, wobei in Formel (I) M^{II} ein oder mehrere zweiwertige Kationen darstellt, und M^{III} ein oder mehrere dreiwertige Kationen darstellt, und x eine Zahl zwischen 0,15 und 0,5 ist, y 1 oder 2 ist, n eine Zahl von 1 bis 10 ist und A ein Anion ausgewählt aus der Gruppe bestehend aus Cl⁻, Br⁻, NO₃⁻, PO₄³⁻, SO₄²⁻, CO₃²⁻, und Kombinationen davon ist.

2. Zubereitung nach Anspruch 1, wobei das kationische Mineral ausgewählt ist aus der Gruppe bestehend aus Calcium-, Chrom-, Germanium-, Eisen-, Cobalt-, Kupfer-, Mangan-, Molybdän-, Vanadium- und Zink-Kationen.

3. Zubereitung nach Anspruch 1 oder 2, wobei das zweiwertige Kation ausgewählt ist aus der Gruppe von zweiwertigen Magnesium-, Chrom-, Zink-, Germanium-, Nickel-, Eisen-, Kupfer, Cobalt-, Calcium-, Molybdän-, Vanadium- und Mangan-Ionen und Kombinationen dieser zweiwertigen Kationen.

4. Zubereitung nach einem der vorhergehenden Ansprüche, wobei das dreiwertige Kation ausgewählt ist aus der Gruppe von dreiwertigen Aluminium-, Chrom-, Eisen-, Cobalt-, Molybdän-, Vanadium- und Mangan-Ionen und Kombinationen dieser dreiwertigen Kationen.

5. Zubereitung nach einem der Ansprüche 3 oder 4, wobei eines oder mehrere der Kationen in der Zubereitung das kationische Mineral bereitstellt oder bereitstellen, an dem oder denen es einem vorgesehenen Patienten mangelt.

6. Zubereitung nach Anspruch 5, wobei M^{II} eine Kombination von Magnesium und zweiwertigem Mineral darstellt, an dem es einem vorgesehenen Patienten mangelt.

7. Zubereitung nach Anspruch 6, wobei das stöchiometrische Verhältnis von Magnesium zu dem zweiwertigen Mangelmineral zwischen 1 : 0 und 0 : 1 liegt.

8. Zubereitung nach Anspruch 7, wobei M^{III} eine Kombination von Aluminium und dreiwertigem Mineral darstellt, an dem es einem vorgesehenen Patienten mangelt.

9. Zubereitung nach Anspruch 8, wobei das stöchiometrische Verhältnis von Aluminium zu dem dreiwertigen Mangelmineral zwischen 1 : 0 und 0 : 1 liegt.

10. Zubereitung nach einem der vorhergehenden Ansprüche, die ferner Antioxidans zur Verhinderung von unerwünschter Oxidation des zweiwertigen Kations zu dreiwertigen Kationen umfasst.

11. Zubereitung nach einem der vorhergehenden Ansprüche, wobei das zweiwertige Kation Fe²⁺ ist.

12. Zubereitung nach einem der Ansprüche 10 oder 11, wobei das Antioxidans in das Schicht-Doppelhydroxid eingeschlossen ist.

13. Zubereitung nach einem der Ansprüche 10 bis 12, wobei das Antioxidans ausgewählt ist aus der Gruppe bestehend aus Ascorbinsäure (Vitamin C) und Chelatisierungsmitteln.

14. Nahrungsergänzungsmittel, Nahrungsmittel oder Futterprodukt zur Behandlung eines Mangels an kationischem Material, das die Zubereitung gemäß einem der Ansprüche 1 bis 13 umfasst.

15. Medizinische Zusammensetzung zur Behandlung eines Mangels an kationischem Material, das die Zubereitung gemäß einem der Ansprüche 1 bis 13 umfasst.

## Revendications

1. Préparation destinée au traitement d'une carence d'un minéral cationique, ladite préparation comprenant un double hydroxyde lamellaire qui contient un ou plusieurs minéraux cationiques, ledit double hydroxyde lamellaire ayant pour formule
[(M^{II})₍₁₋ₓ) (M^{III})ₓ (OH)₂] [A_{x/y}^{y-}·n H₂O) (I),
dans laquelle, dans la formule (I), M^{II} représente un ou plusieurs cations divalents, M^{III} représente un ou plusieurs cations trivalents, x est un nombre valant de 0,15 à 0,5, y vaut 1 ou 2, n est un nombre valant de 1 à 10, et A est un anion choisi dans le groupe constitué par Cl⁻, Br⁻, NO₃⁻, PO₄³⁻, SO₄²⁻, CO₃²⁻ et des combinaisons de ceux-ci.

2. Préparation selon la revendication 1, dans laquelle le minéral cationique est choisi dans le groupe constitué par des cations de calcium, chrome, germanium, fer, cobalt, cuivre, manganèse, molybdène, vanadium et zinc.

3. Préparation selon la revendication 1 ou la revendication 2, dans laquelle le cation divalent est choisi dans le groupe des ions divalents de magnésium, chrome, zinc, germanium, nickel, fer, cuivre, cobalt, calcium, molybdène, vanadium et manganèse et des combinaisons de ces cations divalents.

4. Préparation selon l'une quelconque des revendications précédentes, dans laquelle le cation trivalent est choisi dans le groupe des ions trivalents d'aluminium, chrome, fer, cobalt, molybdène, vanadium et manganèse et des combinaisons de ces cations trivalents.

5. Préparation selon l'une quelconque des revendications 3 ou 4, dans laquelle un ou plusieurs desdits cations contenus dans ladite préparation fournissent le minéral cationique pour lequel un patient cible présente une carence.

6. Préparation selon la revendication 5, dans laquelle M^{II} représente une combinaison de magnésium et d'un minéral divalent pour lequel un patient cible présente une carence.

7. Préparation selon la revendication 6, dans laquelle le rapport stoechiométrique du magnésium sur le minéral divalent carencé se situe dans la plage de 1:0 à 0:1.

8. Préparation selon la revendication 7, dans laquelle M^{III} représente une combinaison d'aluminium et d'un minéral trivalent pour lequel un patient cible présente une carence.

9. Préparation selon la revendication 8, dans laquelle le rapport stoechiométrique du magnésium sur le minéral trivalent carencé se situe dans la plage de 1:0 à 0:1.

10. Préparation selon l'une quelconque des revendications précédentes, comprenant en plus un antioxydant destiné à empêcher une oxydation indésirable desdits cations divalents en cations trivalents.

11. Préparation selon l'une quelconque des revendications précédentes, dans laquelle le cation divalent est le Fe²⁺.

12. Préparation selon l'une quelconque des revendications 10 ou 11, dans laquelle ledit antioxydant est incorporé dans ledit double hydroxyde lamellaire.

13. Préparation selon l'une quelconque des revendications 10 à 12, dans laquelle ledit antioxydant est choisi dans le groupe constitué par l'acide ascorbique (vitamine C) et des agents chélatants.

14. Complément alimentaire, produit alimentaire humain ou animal destiné à traiter une carence d'un minéral cationique, comprenant la préparation selon l'une quelconque des revendications 1 à 13.

15. Composition médicale destinée à traiter une carence d'un minéral cationique, comprenant la préparation selon l'une quelconque des revendications 1 à 13.
